# EUROPÄISCHE PATENTSCHRIFT

(11) **EP 0 981 743 B1**
(45) Veröffentlichungstag und Bekanntmachung des Hinweises auf die Patenterteilung: **28.08.2002**
(21) Anmeldenummer: 99907525.2
(22) Anmeldetag: 09.02.1999
(51) Int. Cl.: G01N 33/00

(54) **Anordnung zur Nullpunktstabilisierung und Kühlung eines Abgassensors**
System for stabilizing the zero-point and cooling an exhaust gas sensor
Système de stabilisation du point zéro et de réfrigération d'un détecteur de gaz brulés

(30) Priorität: 13.03.1998 DE 19810973
(43) Veröffentlichungstag der Anmeldung: 01.03.2000
(73) Patentinhaber: FEV Motorentechnik GmbH, 52078 Aachen (DE)
(72) Erfinder: LEPPERHOFF, Gerhard, D-52223 Stolberg (DE); MEYERDIERKS, Dietrich, D-67098 Bad Dürkheim (DE)
(74) Vertreter: Langmaack, Jürgen, Dipl.-Ing.
(86) Internationale Anmeldenummer: EP9900840
(87) Internationale Veröffentlichungsnummer: WO99047915

(56) Entgegenhaltungen:
- EP-A- 0 719 918
- DE-A- 3 939 166
- DE-A- 4 217 893
- US-A- 4 532 013
- US-A- 4 590 789

## Beschreibung

Um den Schadstoffanteil in Abgasen von Verbrennungsmotoren und Feuerungsanlagen, insbesondere Kleinfeuerungsanlagen zu reduzieren, ist es erforderlich, die Abgaszusammensetzung zu überwachen und über entsprechende Regelungseinrichtungen die Brennstoffumsetzung zu beeinflussen. Bei Verbrennungsmotoren sind zusätzlich noch Abgasnachbehandlungseinrichtungen vorgesehen. Bei Verbrennungsmotoren mit wechselnder Lastanforderung, insbesondere bei Verbrennungsmotoren in Kraftfahrzeugen, ist es erforderlich, die Abgasnachbehandlungseinrichtung in Abhängigkeit von der Lastanforderung und dem daraus resultierenden unterschiedlichen Gehalt an Schadstoffen, insbesondere Stickoxiden und Kohlenwasserstoffen nachzuregeln.

Um derartige Regelungen durchzuführen, werden Abgassensoren verwendet, die jedoch einer Umgebung mit wechselnder Gaszusammensetzung, aber auch mit hohen Temperaturschwankungen und auch hohen Temperaturgradienten ausgesetzt sind. Da die Abgassensoren einen eingeschränkten Arbeitsbereich hinsichtlich einer oberen Temperaturgrenze aufweisen und auch einer sogenannten Drift unterliegen, d. h. infolge von wechselnden Temperatur- und Konzentrationseinflüssen bleibt der Nullpunkt des Abgassensors nicht stabil.

Es sind Verfahren zum Kalibrieren von Meßeinrichtungen bekannt:

DE-A-42 17 893 beschreibt ein Verfahren zur Kalibrierung von Gassensoren für Rauchgasanalysen dergestalt, daß zur Kalibrierung des Meßwertes für die O₂-Konzentrationsbestimmmung als Referenz- oder Kalibriergas Luft benutzt wird.

DE-A-39 39 166 beschreibt auch die Kalibrierung von Sensoren, in diesem Fall unter Verwendung von elektrochemischen Effusionszellen. Auch hierbei geht es um die Einstellung des Konzentrationssignals mit Hilfe einer Kalibriervorrichtung.

In DE-A-35 46 409 wird zur Kalibrierung oder Eichung von chemischen Sensoren ein Eichgas dem in der zu analysierenden Flüssigkeit steckenden Sensor unter Druck zugeführt. Das Eichgas verdrängt dabei die Flüssigkeit am Sensor, wobei durch Erfassung von Druck und Temperatur am Sensor die jeweilige Konzentration der zu eichenden Komponente im Gas rechnerisch erfaßt und mit dem Sensorsignal verglichen werden kann.

In DE-A-31 26 647 wird eine quasi-elektrische Kalibrierung beschrieben, in dem ein elektrischer Widerstand benutzt wird, um durch Abgleich dieses Konstantwiderstandes mit den simulierten Meßwerten die Kalibrierung des Meßgerätes mit Kalibriergasen zeitlich zu dehnen.

Die vorbekannten Verfahren setzen jedoch durchgehend das Vorhandensein eines Meßwertes oder einer Meßwertanzeige voraus, also immer eines Wertes oberhalb des Nullpunktes, da nur dann eine Abweichung feststellbar ist. Das "Auswandern" oder "Driften" des Nullpunktes ist mit den bekannten Verfahren nicht feststellbar und kann somit bei einer Regeleinrichtung auch nicht berücksichtigt werden.

US-A-4,590,789 zeigt einen Abgassensor, der ein Gehäuse mit zwei durch eine Ventil/Scheidewand getrennte Enden umfaßt. Das erste Ende, in dem ein Sensor eingebaut ist, ist mit einem Deckel verschließbar, der mit der Gaszufuhrleitung verbunden ist. Das zweite Ende ist offen und weist mehrere Öffnungen zur Einführung von Luft auf. Nach der Abgasmessung kann die Ventil/Scheidewand aus einer "Zu"-Position in eine "Auf"-Position gestellt werden, um einen Spülung des Sensors mit Umgebungsluft zu erreichen.

US-A-4,532,013 zeigt eine Anordnung an einer Kolbenbrennkraftmaschine, bei der zur Überprüfung eines Abgassensors, der von Zeit zu Zeit der Umgebungsluft ausgesetzt ist und die so enthaltene Messung mit einem auf einem Mikroprozessor gespeicherten Standardwert verglichen wird. Die Luft wird mittels Gebläse zugeführt.

Eine Möglichkeit, um die Drift zu erfassen und auch eine Anzeigestabilität zu erreichen, besteht darin, mit Hilfe verschiedener Algorithmen in der Software der Regeleinrichtung die wechselnden Einflüsse zu berücksichtigen. Hierfür fehlt jedoch ein unabhängiges Bezugssystem, das auch eine gewisse Alterungsstabilität aufweist.

Der Erfindung liegt somit die Aufgabe zugrunde, ein Anordnung zu schaffen, durch die die Drift eines Abgassensors auskompensiert werden kann und auch bei entsprechend hohen Abgastemperaturen der Arbeitstemperaturbereich des Abgassensors eingehalten werden kann.

Diese Aufgabe wird erfindungsgemäß gelöst durch eine Anordnung zur Nullpunktstabilisierung eines Abgassensors an einer Einrichtung zur thermischen Energieumsetzung von gasförmigen Brennstoffen mit den Merkmalen des Anspruchs 1. Dadurch, daß der Abgassensor periodisch wechselnd vom Abgas und von Frischluft umspült wird, ist dem Abgassensor ein Bezugssystem vorgegeben, da er periodisch immer wieder von Frischluft umspült wird, so daß dementsprechend in dieser Phase der gleiche Ausgangswert, d. h. der "Nullpunkt" angezeigt werden muß. Infolge von Alterungseinflüssen, Temperatureinflüssen oder dergl. kann sich jedoch das als Nullpunktsignal zu wertende Signal verändern, d. h. driften, so daß mit der erfindungsgemäßen Vorrichtung in der Regeleinrichtung die Möglichkeit besteht, diese Drift während der Phase der Frischluftumspülung durch einen Vergleich mit dem vorgegebenen Wert zu erfassen und dann über die Regeleinrichtung auszukorrigieren. Damit ist mit der Vorrichtung die Möglichkeit gegeben, auch während des Betriebes den Abgassensor und die zugehörige Regeleinrichtung laufend auf den Nullpunkt zurückzuführen. Bei entsprechender Temperatur der Frischluft, beispielsweise Umgebungstemperatur, wird zudem der Abgassensor gekühlt und kann entsprechend der Temperaturdifferenz in seinem Arbeitstemperaturbereich gehalten werden.

Der Begriff "Einrichtung zur thermischen Energieumsetzung" im Sinne der vorliegenden Erfindung umfaßt neben Verbrennungsmotoren, hier insbesondere Kolbenmotoren, auch Feuerungsanlagen, insbesondere Kleinfeuerungsanlagen. Der Begriff "gasförmige Brennstoffe" umfaßt sowohl Brennstoffe, die von Haus aus in Gasform vorliegen, wie sie beispielsweise für Kleinfeuerungsanlagen verwendet werden, aber auch flüssige Brennstoffe, die für die Energieumsetzung zerstäubt und/oder verdampft werden.

Der durch die Anordnung einer steuerbaren Ventileinrichtung mögliche Wechsel zwischen Abgasumspülung und Frischluftumspülung erlaubt es, sowohl in Abhängigkeit vom Einsatzfall als auch mit Rücksicht auf wechselnde Lastfälle die Zeitdauer der Abgasumspülung und der Frischluftumspülung, aber auch die Häufigkeit des Wechsels zwischen beiden Beaufschlagungszuständen zu beeinflussen.

So wird man beispielsweise bei Kleinfeuerungsanlagen, die einen über die jeweilige Betriebszeit im wesentlichen konstanten Brennstoffdurchsatz aufweisen, nur mit geringer Häufigkeit eine Frischluftumspülung vornehmen müssen. Die Vorrichtung kann dann auch noch so mit der Regeleinrichtung verkoppelt werden, daß ein Wechsel zwischen Abgasumspülung und Frischluftumspülung nur dann vorgenommen wird, wenn auch tatsächlich Brennstoff umgesetzt wird. Demgegenüber wird zweckmäßig bei einem Verbrennungsmotor, insbesondere bei einem Fahrzeugmotor mit seinen wechselnden Lastanforderungen und den daraus resultierenden wechselnden Anteilen an Schadstoffen im Abgas und nicht zuletzt wegen der sehr viel höheren Abgastemperatur ein häufiger Wechsel zwischen Abgas-umspülung und Frischluftumspülung vorgenommen werden müssen. Damit ist nicht nur sichergestellt, daß über den Abgassensor für die nachgeordnete Regeleinrichtung immer die "aktuellen" Abgasdaten zur Verfügung gestellt werden, sondern darüber hinaus auch noch während der Periode der Frischluftumspülung eine Kühlung des Abgassensors erreicht wird, so daß dieser in seinem Arbeitstemperaturbereich gehalten werden kann.

Dadurch, daß der Abgassensor über die Zweigleitung, die auch kleinere Strömungsquerschnitte im Umströmungsbereich für den Abgassensor zur Folge hat, mit einem aus dem Abgasstrom abgezweigten Teilstrom umspült wird, kann nicht nur ein einwandfreies Strömungsbild im Kontaktbereich mit dem Abgassensor bewirkt werden, sondern der Teilstrom kann auch aus einem Kernbereich des Abgasstroms abgegriffen werden, so daß Störeinflüsse weitgehend ausgeschaltet werden können.

Während es grundsätzlich möglich ist, die Frischluft über ein Zusatzgebläse im Umspülungsbereich des Abgassensors zuzuführen, bietet die erfindungsgemäße Anordnung auch die Möglichkeit, daß die Frischluft unter Ausnutzung eines durch den Druck im Abgasstrom vorgegebenen Druckgefälles dem Abgassensor zugeführt wird. Dies ist insbesondere zweckmäßig bei Kleinfeuerungsanlagen, da im Abgaskamin ein ausreichender Unterdruck gegenüber der Umgebungsluft vorhanden ist, so daß die Frischluft durch den Unterdruck im Abgaskamin in den Umspülungsbereich des Abgassensors durch ein natürliches Druckgefälle eingesaugt wird.

Bei einem Einsatz an einer durch eine Brennkraftmaschine, insbesondere einer Kolbenbrennkraftmaschine gebildeten Einrichtung zur thermischen Umsetzung, die eine Saugleitung für die Verbrennungsluft und eine Abgasleitung aufweist, kann der Abgassensor periodisch mit einem aus der Abgasleitung abgezweigten Abgasteilstrom und einem aus der Umgebung angesaugten Frischluftstrom umspült werden und beide Gasströme in die Saugleitung jeweils eingeführt werden. Hierbei wird in vorteilhafter Weise das bestehende Druckgefälle zwischen der Abgasleitung einerseits, die einen Überdruck aufweist und der Saugleitung andererseits, die gegenüber der Umgebungsluft einen geringen Unterdruck aufweist, zur Förderung sowohl des Abgasteilstroms als auch des Frischluftstroms ausgenutzt.

Für alle Anwendungsfälle kann das steuerbare Ventil mit einem Stellantrieb verbunden sein, der über eine vorgeschaltete Regeleinrichtung oder Steuerungseinrichtung angesteuert wird. Zweckmäßig ist es jedoch, wenn das steuerbare Ventil selbsttätig über einen wechselnden, insbesondere einen pulsierenden Gasdruck in der Abgasleitung angesteuert wird. Dieser Anwendungsfall ist insbesondere bei Kolbenbrennkraftmaschinen vorteilhaft, da hier ein pulsierender Abgasstrom vorliegt, der dann zur Betätigung der Ventileinrichtung ausgenutzt werden kann. Die Überdruckwelle öffnet den Zustrom von Abgas und verschließt gleichzeitig den Zustrom von Frischluft, während die Unterdruckwelle den Zustrom des Abgases schließt und gleichzeitig den Zustrom der Frischluft öffnet. Damit wird nicht nur eine wechselnde Umspülung des Abgassensors mit Abgas und Frischluft erreicht, sondern zugleich auch der Abgassensor auf einer mittleren Arbeitstemperatur gehalten.

Bei Kleinfeuerungsanlagen kann die Anordnung unmittelbar im Kaminbereich verwirklicht werden, da der Kaminzug ausreicht, um beim Öffnen des Frischlufteinlasses aus der Umgebung Frischluft in die Zweigleitung anzusaugen.

Bei einer Einrichtung zur thermischen Energieumsetzung in Form einer Brennkraftmaschine mit einem Luftansaugkanal und einem Abgaskanal die Zweigleitung durch eine Verbindungsleitung zwischen dem Abgaskanal und dem Luftansaugkanal gebildet wird, wobei der Abgassensor in der Verbindungsleitung angeordnet ist, ist es möglich, ohne zusätzliche Fördereinrichtungen für den Abgasteilstrom und/oder den Frischluftteilstrom das bestehende natürliche Gefälle zwischen dem Abgaskanal und dem Luftansaugkanal auszunutzen und zwar sowohl zur Förderung des Abgasteilstroms als auch des Frischluftstroms.

In Ausgestaltung der Erfindung ist insbesondere für den Einsatz an Kolbenbrennkraftmaschinen vorgesehen, daß die Ventileinrichtung eine Sperrkammer aufweist, die in eine mit der Abgasleitung verbundene Abgaskammer und eine über die Verbindungsleitung mit der Saugleitung verbundenen Luftkammer unterteilt ist, daß die Abgaskammer einen Gaseinlaß und die Luftkammer einen Frischlufteinlaß aufweist und daß eine Ventilanordnung vorgesehen ist, durch die der Gaseinlaß und der Frischlufteinlaß abwechselnd verschließbar sind.

Die Ventilanordnung kann beispielsweise einen doppeltwirkenden elektromagnetischen Aktuator zur Betätigung für einen Stellantrieb aufweisen.

Insbesondere für die Verwendung an einer Kolbenbrennkraftmaschine kann eine Ventilanordnung vorgesehen sein, die eine Ventilmembran aufweist, die die Sperrkammer in die Abgaskammer und die Luftkammer unterteilt und durch die periodisch die Mündung des Frischlufteinlasses unter der Einwirkung des pulsierenden Abgasstromes verschließbar ist.

Weitere vorteilhafte Ausgestaltungen der Erfindung sind in den Unteransprüchen angegeben.

Die Erfindung wird anhand schematischer Zeichnungen von Ausführungsbeispielen näher erläutert. Es zeigen:
- Fig. 1: eine Kleinfeuerungsanlage,
- Fig. 2: eine Kolbenbrennkraftmaschine,
- Fig. 3: eine Ventilanordnung mit Stellantrieb,
- Fig. 4: eine durch Pulsation des Abgasstroms betätigte Ventilanordnung.

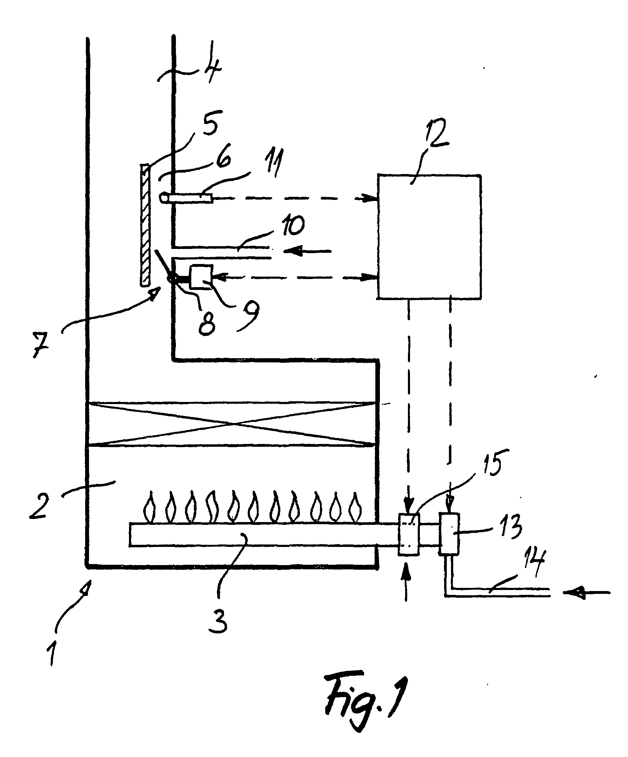

In Fig. 1 ist eine Kleinfeuerungsanlage, beispielsweise eine Gasheizungsanlage dargestellt, deren Heizkessel 1 einen Brennraum 2 aufweist, in dem beispielsweise ein atmosphärischer Brenner 3 angeordnet ist. Die Gase werden aus dem Feuerungsraum 2 über einen Kamin 4 nach außen abgeführt.

Im Kamin 4 ist durch einen entsprechenden Einbau 5 eine zweigleitung 6 angeordnet, die an ihrem Einlaß 7 mit Ventileinrichtung 8, beispielsweise einer Stellklappe versehen ist, die über einen Stellantrieb 9 betätigbar ist. Mit Hilfe der Ventileinrichtung 8kann der Einlaßbereich 7 abgesperrt werden, so daß kein Abgas durch die Zweigleitung 6 hindurchströmen kann.

Im Einlaßbereich 7 mündet in die Zweigleitung 6 eine Frischluftleitung 10 ein, deren Einmündung durch die Klappe der Ventileinrichtung 8 bei entsprechender Klappenstellung ebenfalls verschließbar ist.

In Strömungsrichtung der Abgase in der Zweigleitung 6 gesehen mit Abstand hinter dem Einlaßbereich 7 und dem Frischlufteinlaß ist ein Abgassensor 11 angeordnet.

Als Abgassensor 11 kann beispielsweise ein Mischoxidsensorelement eingesetzt werden, durch das in gleicher Weise reduzierende und oxidierende Gasanteile erfaßt werden können. Anstelle eines derartigen Mehrfachsensors ist es auch möglich, jeweils für die reduzierenden und für die oxidierenden Gasanteile einen gesonderten Sensor einzusetzen.

Die Heizeinrichtung ist mit einer Regeleinrichtung 12 versehen, über die entsprechend den Anforderungen des Wärmeverbrauches die Gaszufuhr zum Brenner 3 beispielsweise über eine Stelleinrichtung 13 in der Gaszufuhrleitung 14 verändert wird. Über die Regeleinrichtung 12 kann ferner über eine Stelleinrichtung 15 auf die Zufuhr von Primärluft und von Sekundärluft zum Brenner 3 Einfluß genommen werden, entsprechend den Vorgaben durch den auf die Regeleinrichtung 12 aufgeschalteten Abgassensor 11.

Über die Regeleinrichtung 12 kann nun über ein beispielsweise zeitgesteuertes Signal die Ventileinrichtung 8 betätigt werden, so daß nach einer längeren Öffnungsperiode für den Abgasteilstrom und bei geschlossenem Frischlufteinlaß 10 die Umspülung des Abgassensors 11 mit Abgas gesperrt wird, so daß über einen ebenfalls vorgebbaren, kürzeren Zeitraum der Abgassensor 11 mit Frischluft umspült wird. Das in der Phase der Frischluftumspülung vom Abgassensor 11 abgegebene Signal wird in der Regeleinrichtung 12 mit einem vorgegebenen Sollwert verglichen. Sofern hier durch Driftvorgänge, beispielsweise durch Alterungsprozesse eine Signalabweichung feststellbar ist, wird automatisch in der Regeleinrichtung eine entsprechende "Nullpunktkorrektur" vorgenommen. Über die Regeleinrichtung 12 ist zweckmäßig zugleich sichergestellt, daß das Umspülen mit Frischluft immer nur dann erfolgt, wenn der Brenner 3 in Betrieb ist, so daß für die Nullpunktkalibrierung sichergestellt ist, daß unmittelbar vor dem Umspülen mit Frischluft der Abgassensor 11 auch mit Abgas umspült war.

Wird beispielsweise, wie vorstehend beschrieben, bei einer Feuerungsanlage ein atmosphärischer Brenner eingesetzt, kann in den Stillstandsphasen des Brenners 3, in denen aus Sicherheitsgründen Luft durch die Abgasleitung, beispielsweise durch den Kamin 4 geführt wird, diese Luft zur Nullpunktstabilisierung genutzt werden. Die Anwendung des Verfahrens bei Feuerungsanlagen ist nicht auf Feuerungsanlagen mit einem atmosphärischen Brenner beschränkt.

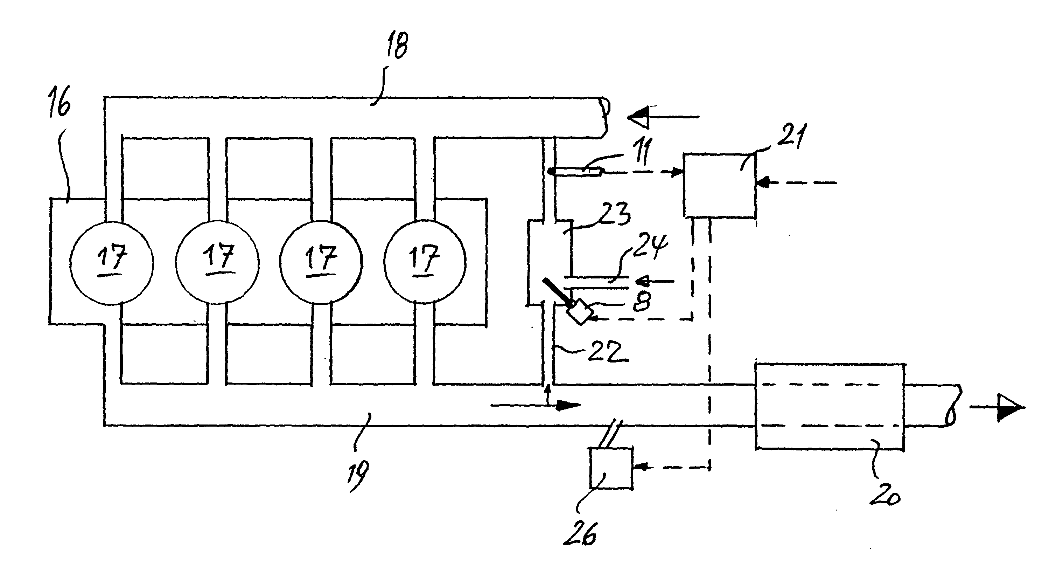

In Fig. 2 ist als weiterer Anwendungsfall eine Vier-Zylinder-Kolbenbrennkraftmaschine dargestellt, deren Zylinder 17 einerseits mit einem Luftansaugkanal 18 und andererseits mit einem Abgaskanal 19 verbunden sind. Im Abgaskanal 19 ist eine Abgasnachbehandlungseinrichtung 20 angeordnet, die beispielsweise einen reduzierenden und/oder einen oxidierenden Abgasbehandlungsteil aufweisen kann.

Die Kolbenbrennkraftmaschine 16 steht mit einer Motorsteuerung 21 in Verbindung, über die entsprechend den Lastanforderungen in üblicher Weise die Motorfunktionen wie Zündung, Kraftstoffzufuhr, beispielsweise Kraftstoffeinspritzung usw. geregelt und gesteuert werden.
Bei einer derartigen Kolbenbrennkraftmaschine müßte ein Abgassensor im Abgaskanal 19 angeordnet sein. Das Problem besteht jedoch darin, daß die Abgase einer Brennkraftmaschine sehr heiß sind, daß andererseits die bisher üblichen Abgassensoren mit ihren Arbeitstemperaturen unterhalb der Abgastemperaturen liegen, so daß bisher nur das Luftverhältnis im Abgas durch eine Lambdasonde erfaßt werden konnte. Die Signale des Abgassensors sollen zur Steuerung der Abgasemission über die Motorsteuerung 21 dienen, dergestalt, daß bei einer Abgasbehandlung durch Speicherkatalysatoren die Emissionen hinter dem Katalysator 20 mit einem Abgassensor 11 gemessen werden. Übersteigen die Konzentrationen einen kritischen Wert, wird die Regeneration des Speicherkatalysators durch Anfettung des Gemisches geleitet.

In Fig. 2 ist eine Anordnung dargestellt, bei der beispielsweise zur Stickoxidreduzierung entsprechende Reduktionsmittel über einer Dosiereinrichtung 26 dem Abgas zugegeben werden oder durch eine Regelung der Abgaszusammensetzung mit Hilfe der Motorsteuereinrichtung 21 ein zur Noₓ-Reduzierung erforderliches HC/Noₓ-Verhältnis eingestellt wird. Dazu ist der Abgassensor 11 mit der Verbindungsleitung 22 in Strömungsrichtung vor der Abgasnachbehandlungseinrichtung 20 anzuordnen. Im Falle einer Motorregelung bzw. Abgasnachbehandlungseinrichtungsregelung über die Motorsteuerung 21 mit Beeinflussung der Abgaszusammensetzungskonzentrationen durch die Zugabe von Reduktionsmitteln, beispielsweise bei Speicherkatalysatoren ist der Anschluß der Verbindungsleitung 22 hinter der Abgasnachbehandlungseinrichtung anzuordnen.

Erfindungsgemäß ist nun als Zweigleitung eine Verbindungsleitung 22 zwischen dem Abgaskanal 19 und dem Luftansaugkanal 18 vorgesehen, die möglichst unmittelbar hinter dem in Strömungsrichtung der Abgase gesehen letzten der Zylinder 17 einmündenden Abgasauslaß abzweigt oder in Abhängigkeit von der Abgasnachbehandlungseinrichtung 20 nach dieser abzweigt. Zur Verbessung der Signaldynamik des Abgassensors 11 ist der Abgassensor 11 mit Sperrkammer 23 und Frischluftzufuhr 24 direkt auf der Abgasleitung 19 angeordnet.

In der Verbindungsleitung 22 ist eine Sperrkammer 23 vorgesehen, die einerseits mit der Abgasleitung 19 und andererseits mit dem Luftansaugkanal 18 verbunden ist und in die ein Frischlufteinlaß 24 einmündet. Durch eine steuerbare Ventileinrichtung 8 kann nun wechselnd der Einlaß von Abgas und von Frischluft in die Sperrkammer 23 bewirkt werden, wo die Druckdifferenz zwischen dem pulsierenden Abgasstrom im Abgaskanal 19 und der demgegenüber geringe Unterdruck im Luftansaugkanal 18 ausreicht, um sowohl aus dem Abgaskanal 19 als auch über den Lufteinlaß 24 Abgas bzw. Luft über die Sperrkammer 23 in den Luftansaugkanal 18 strömen zu lassen. Über die Ventileinrichtung 8.1 kann nun jeweils die Zeitdauer bestimmt werden, in der der Sperrkammer Abgas oder Frischluft zugeführt wird.

In Strömungsrichtung der Gase gesehen hinter der Sperrkammer 23 ist der Abgassensor 11 angeordnet, über den die Zusammensetzung des Abgases erfaßt werden kann. Der Abgassensor 11 ist wiederum auf eine Regeleinrichtung aufgeschaltet, die beispielsweise auch Teil der Motorsteuerung 21 sein kann und über die zum einen die "Nullpunkterfassung" des Abgassensors 11 bei Frischluftbeaufschlagung und die Erfassung der Abgaszusammensetzung bei Abgasbeaufschlagung erfaßt werden kann. Je nach der Gaszusammensetzung kann dann über einen entsprechenden Regeleingriff, beispielsweise die Zufuhr von reduzierenden Stoffen über eine entsprechende Zugabeeinrichtung 26 in den Abgaskanal 19 vor der Nachbehandlungseinrichtung 20 bewirkt werden. Oder aber es können entsprechende Regeleingriffe über die Motorsteuerung 21 vorgenommen werden.

Da der Abgassensor 11 immer wieder mit verhältnismäßig kühler Frischluft beaufschlagt wird, stellt sich am Abgassensor eine Temperatur unter der Abgastemperatur ein, so daß der Abgassensor 11 in seinem Arbeitstemperaturbereich gehalten werden kann.

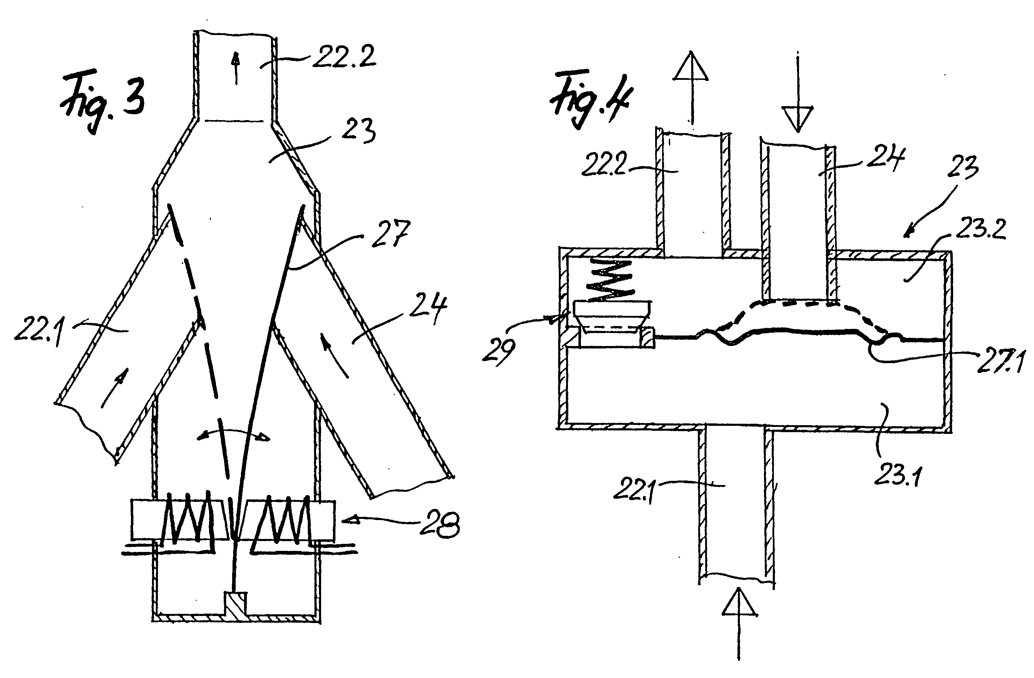

In Fig. 3 ist ein Ausführungsbeispiel für eine Sperrkammer 23 dargestellt. In die Sperrkammer 23 mündet der mit dem Abgaskanal 19 verbundene Teil der Verbindungsleitung 22.1 sowie der Lufteinlaß 24 ein. Die Mündung der Verbindungsleitung 22.1 sowie die Mündung des Lufteinlasses 24 sind einander gegenüberliegende anordnet, so daß über eine federnde Ventilplatte 27, die beispielsweise über einen elektromagnetischen Aktuator 28 hin und her schwenkbar ist, entsprechend der Ansteuerung der Abgasleitung 22.1 und der Lufteinlaß 24 geöffnet bzw. geschlossen werden kann. Der jeweils in die Sperrkammer 23 eintretende Gasstrom kann dann über den mit dem Luftansaugkanal 18 verbundenden Teil 22.2 der Verbindungsleitung abfließen und am Abgassensor 11 vorbeiströmen. Die Ansteuerung des elektromagnetischen Aktuators 28 kann beispielsweise über die Motorsteuerung 21 erfolgen.

Fig. 4 zeigt eine selbsttätig arbeitende Ventileinrichtung. Bei dieser Ausgestaltung ist wiederum eine Sperrkammer 23 vorgesehen, die durch eine Ventilmembran 27.1 in eine Abgaskammer 23.1 und eine Luftkammer 23.2 unterteilt ist. In die Abgaskammer 23.1 mündet wiederum der mit dem Abgaskanal 19 verbundene Teil 22.1 der Verbindungsleitung ein, während in die Luftkammer 23.2 der Lufteinlaß 24 einmündet. Die Luftkammer 23.2 steht ferner mit dem zum Luftansaugkanal 18 führenden Teil 22.2 der Verbindungsleitung 22 in Verbindung.

In der Sperrkammer 23 ist ferner ein Rückschlagventil 29 angeordnet, das so eingestellt ist, daß es bei maximalem Überdruck im pulsierenden Abgasstrom öffnet, so daß aus der Abgaskammer 23.1 Abgas in die Luftkammer 23.2 übertreten kann.

Die Ventilmembran 27.1 ist so ausgebildet, daß sie in gleicher Weise bei Überdruck im Abgasstrom die Einmündung des Lufteinlasses 24 verschließt, und gleichzeitig Abgas aus der Abgasleitung 23.1 über das dann geöffnete Rückschlagventil 29 durch die Luftkammer 23.2 in die zum Luftansaugkanal 18 führende Verbindungsleitung 22.2 einströmen kann. Die dem den Lufteinlaß 24 verschließenden Gasauslaßstoß nachfolgende Unterdruckwelle im Abgaskanal 19 läßt die Ventilmembran 27.1 zurückspringen, so daß der Lufteinlaß 24 geöffnet und das Rückschlagventil 29 verschlossen wird und aufgrund der Druckdifferenz zwischen der Umgebungsluft und dem Luftansaugkanal 18 Frischluft durch die Luftkammer 23.2 in die Zweigleitung 22.2 strömen kann.

Bei beiden Ausführungsbeispielen ist der Abgassensor 11 in dem zum Saugkanal 18 führenden Teil 22.2 der Verbindungsleitung 22 angeordnet. Räumlich kann die Anordnung des Systems auch noch so getroffen werden, daß zumindest der Teil 22.2 der Verbindungsleitung 22 im Kühlluftstrom liegt, so daß zusätzlich zu der direkten Frischluftbeaufschlagung des Abgassensors 11 auch der Umgebungsbereich des Abgassensors gekühlt wird und so das Temperaturniveau im unmittelbaren Bereich des Abgassensors niedrig gehalten werden kann.

## Patentansprüche

1. Anordnung zur Nullpunktstabilisierung und Kühlung eines Abgassensors (11) einer Regeleinrichtung (12, 21) zur Verminderung der Schadstoffanteile im Abgas einer Einrichtung (1; 16) zur thermischen Energieumsetzung mittels gasförmiger Brennstoffe, die zumindest einen Abgaskanal (4; 19) aufweist, der mit einer Zweigleitung (6; 22) versehen ist, die einen Abgaseinlaß (7; 22.2) und einen Frischlufteinlaß (10; 24) aufweist und in der in Strömungsrichtung der Gase hinter den Einlässen der Abgassensor (11) angeordnet ist und daß im Einmündungsbereich der Einlässe eine Ventileinrichtung (8) angeordnet ist, durch die die beiden Einlässe abwechselnd verschließbar sind.

2. Anordnung nach Anspruch 1, **dadurch gekennzeichnet, daß** bei einer Einrichtung zur thermischen Energieumsetzung in Form einer Brennkraftmaschine (16) mit einem Luftansaugkanal (18) und einem Abgaskanal (19) die Zweigleitung durch eine Verbindungsleitung (22) zwischen dem Abgaskanal (19) und dem Luftansaugkanal (18) gebildet wird und daß der Abgassensor (11) in der Verbindungsleitung (22) angeordnet ist.

3. Anordnung nach Anspruch 1 oder 2, **dadurch gekennzeichnet, daß** die Ventileinrichtung (8) durch ein steuerbares, mit einem Stellantrieb versehenes Ventil gebildet wird.

4. Anordnung nach einem der Ansprüche 1 bis 3, **dadurch gekennzeichnet, daß** die Ventileinrichtung eine Sperrkammer (23) aufweist, die in eine mit der Abgasleitung (9) verbundene Abgaskammer (23.1) und eine über die Verbindungsleitung (22.3) mit dem Luftansaugkanal (18) verbundene Luftkammer (23.2) unterteilt ist, daß die Abgaskammer (23.1) einen Abgaseinlaß und die Luftkammer (23.2) einen Frischlufteinlaß (24) aufweist und daß eine Ventilanordnung (8.1) vorgesehen ist, durch die der Gaseinlaß und der Frischlufteinlaß abwechselnd verschließbar sind.

5. Anordnung nach einem der Ansprüche 1 bis 4, **dadurch gekennzeichnet, daß** die Ventilanordnung (8) durch eine Ventilmembran (27.1) gebildet wird, die die Sperrkammer (23) in die Abgaskammer (23.1) und die Luftkammer (23.2) unterteilt und durch die periodisch die Mündung des Frischlufteinlasses (24) verschließbar ist und daß zwischen der Abgaskammer (23.1) und der Luftkammer (23.2) ein als Rückschlagventil (29) ausgebildetes Überströmventil für das Abgas angeordnet ist.

6. Anordnung nach einem der Ansprüche 1 bis 5, **dadurch gekennzeichnet, daß** die Ventileinrichtung mit einem steuerbaren Stellantrieb versehen ist.

7. Anordnung nach einem der Ansprüche 1 bis 5, **dadurch gekennzeichnet, daß** das steuerbare Ventil selbsttätig über einen wechselnden, insbesondere pulsierenden Gasdruck in der Abgasleitung ansteuerbar ausgebildet ist.

8. Anordnung nach einem der Ansprüche 1 bis 7, **dadurch gekennzeichnet, daß** die Einrichtung zur thermischen Energieumsetzung durch eine Brennkraftmaschine, insbesondere eine Kolbenbrennkraftmaschin gebildet wird, die eine Saugleitung für die Verbrennungsluft und eine Abgasleitung aufweist, wobei eine Einrichtung vorgesehen ist, die bewirkt, daß der Abgassensor periodisch mit einem aus der Abgasleitung abgezweigten Abgasteilstrom und einem aus der Umgebung angesaugten Frischluftstrom umspült wird und beide Gasströme in die Saugleitung jeweils eingeführt werden.

## Claims

1. An arrangement for stabilising the zero point and cooling an exhaust-gas sensor (11) of a control system (12, 21) for reducing the contents of pollutants in the exhaust gas of an apparatus (1; 16) for the thermal conversion of energy by means of gaseous fuels, which has at least one exhaust-gas duct (4; 19) which is provided with a branch line (6; 22) which has an exhaust-gas inlet (7; 22.2) and a fresh-air inlet (10; 24) and in which the exhaust-gas sensor (11) is arranged in the direction of flow of the gases after the inlets and that a valve means (8) is arranged in the opening region of the inlets, through which means the two inlets can be closed alternately.

2. An arrangement according to Claim 1, **characterised in that** in the case of an apparatus for the thermal conversion of energy in the form of an internal-combustion engine (16) with an air intake duct (18) and an exhaust-gas duct (19) the branch line is formed by a connecting line (22) between the exhaust-gas duct (19) and the air intake duct (18) and that the exhaust-gas sensor (11) is located in the connecting line (22).

3. An arrangement according to Claim 1 or 2, **characterised in that** the valve means (8) is formed by a controllable valve provided with a servo drive.

4. An arrangement according to one of Claims 1 to 3, **characterised in that** the valve means has a blocking chamber (23) which is subdivided into an exhaust-gas chamber (23.1) connected to the exhaust-gas line (9) and an air chamber (23.2) connected to the air intake duct (18) via the connecting line (22.3), that the exhaust-gas chamber (23.1) has an exhaust-gas inlet and the air chamber (23.2) has a fresh-air inlet (24) and that a valve arrangement (8.1) is provided through which the gas inlet and the fresh-air inlet can be closed alternately.

5. An arrangement according to one of Claims 1 to 4, **characterised in that** the valve arrangement (8) is formed by a valve membrane (27.1) which subdivides the blocking chamber (23) into the exhaust-gas chamber (23.1) and the air chamber (23.2) and through which periodically the opening of the fresh-air inlet (24) can be closed and that an overflow valve for the exhaust gas which is in the form of a non-return valve (29) is arranged between the exhaust-gas chamber (23.1) and the air chamber (23.2).

6. An arrangement according to one of Claims 1 to 5, **characterised in that** the valve means is provided with a controllable servo drive.

7. An arrangement according to one of Claims 1 to 5, **characterised in that** the controllable valve is designed to be triggered automatically by means of a changing, in particular pulsating, gas pressure in the exhaust-gas line.

8. An arrangement according to one of Claims 1 to 7, **characterised in that** the apparatus for thermal energy conversion is formed by an internal combustion engine, in particular a piston internal combustion engine, which has a suction line for the combustion air and an exhaust-gas line, wherein a means is provided which causes the exhaust-gas sensor to be flushed periodically with a partial stream of exhaust gas which is branched off from the exhaust-gas line and a fresh-air stream which is drawn in from the surroundings, and causes both gas streams each to be introduced into the suction line.

## Revendications

1. Système de stabilisation du point zéro et de réfrigération d'un détecteur de gaz brûlés (11) d'un dispositif de régulation (12, 21) pour réduire les taux de matières toxiques dans les gaz brûlés d'un dispositif (1, 16) de conversion d'énergie thermique au moyen de combustibles gazeux, qui présente au moins un canal de gaz brûlés (4, 19) doté d'une conduite de dérivation (6, 22), laquelle présente une admission de gaz brûlés (7, 22.2) et une admission d'air frais (10, 24) et dans laquelle le détenteur de gaz brûlés (11) est monté derrière les admissions dans le sens de circulation des gaz, un dispositif de soupape (8) étant agencé dans la zone de l'embouchure des admissions, par lequel les deux admissions peuvent être obturées alternativement.

2. Système selon la revendication 1, **caractérisé par le fait que**, dans le cas d'un dispositif de conversion d'énergie thermique constitué par un moteur à combustion interne (16) avec un canal d'aspiration d'air (18) et un canal des gaz brûlés (19), la conduite de dérivation est formée par une conduite de liaison (22) entre le canal de gaz brûlés (19) et le canal d'aspiration d'air (18), le détecteur de gaz brûlés (11) étant monté dans la conduite de liaison (22).

3. Système selon la revendication 1 ou 2, **caractérisé par le fait que** le dispositif de soupape (8) est constitué par une soupape réglable dotée d'un servomoteur.

4. Système selon une des revendications 1 à 3, **caractérisé par le fait que** le dispositif de soupape présente une chambre d'arrêt (23) qui est divisée en une chambre de gaz brûlés (23.1) reliée à la conduite de gaz brûlés (9) et une chambre d'air (23.2) reliée au canal d'aspiration d'air (18) par la conduite de liaison (22.3), que la chambre de gaz brûlés (23.1) présente une admission de gaz brûlés et la chambre d'air (23.2) une admission d'air frais (24), et qu'il est prévu un système de soupape (8.1) par lequel l'admission de gaz et l'admission d'air frais peuvent être obturées alternativement.

5. Système selon une des revendications 1 à 4, **caractérisé par le fait que** le système de soupape (8) est constitué par une membrane de soupape (27.1) qui divise la chambre d'arrêt (23) en la chambre de gaz brûlés (23.1) et la chambre d'air (23.2) et par laquelle l'embouchure de l'admission d'air frais (24) peut être périodiquement obturée, et qu'une soupape de décharge pour les gaz brûlés est montée sous la forme d'une soupape de non-retour (29) entre la chambre de gaz brûlés (23.1) et la chambre d'air (23.2).

6. Système selon une des revendications 1 à 5, **caractérisé par le fait que** le dispositif de soupape est doté d'un servomoteur réglable.

7. Système selon une des revendications 1 à 5, **caractérisé par le fait que** la soupape réglable est conçue de manière à pouvoir être commandée automatiquement par une pression de gaz changeant, et notamment pulsant, dans la conduite de gaz brûlés.

8. Système selon une des revendications 1 à 7, **caractérisé par le fait que** le dispositif de conversion d'énergie thermique est constitué par un moteur à combustion interne, et notamment un moteur à combustion interne à piston, qui présente une conduite d'aspiration pour l'air de combustion et une conduite de gaz brûlés, et dans lequel il est prévu un dispositif qui assure que le détecteur de gaz brûlés est périodiquement rempli par un courant divisé de gaz brûlés en provenance de la conduite de gaz brûlés et par un courant d'air frais aspiré depuis l'environnement, et que les deux courants gazeux sont introduits dans la conduite d'aspiration.
